# EUROPEAN PATENT APPLICATION

(11) **EP 4 335 347 A2**
(43) Date of publication of application: **13.03.2024**
(21) Application number: 23196398.4
(22) Date of filing: 08.09.2023
(51) Int. Cl.: A47L 13/17, C11D 3/50, C11B 9/00, A61Q 13/00, A61K 8/33, A61K 8/37, A61Q 19/00

(54) **CLEANING IMPLEMENT**

(30) Priority: 08.09.2022 US 202263404635 P
(71) Applicant: The Procter & Gamble Company, Cincinnati, OH 45202 (US)
(72) Inventor: FREEMAN, Natalie Rose, Cincinnati, 45202 (US); ZAVAKOS, Rachel Marie, Cincinnati, 45202 (US)
(74) Representative: P&G Patent Belgium UK

(57) **Abstract**

A cleaning implement includes a foam having a density from 8 to 36 kg/m³. The cleaning implement has a thickness, a width, and a depth. The thickness is at least 22 mm, the width is less than or substantially equal to the depth, and the width is greater than the thickness. Methods for cleaning hard surfaces with a cleaning implement include bringing the cleaning implement into contact with a hard surface.

## Description

### TECHNICAL FIELD

The present disclosure is directed to a cleaning implement including a foam having a density from 8 to 36 kg/m³ and a thickness of at least 22 mm.

### BACKGROUND

Recently, a novel application for melamine-formaldehyde foams in the area of hard surface cleaning has been identified. Cleaning implements of cut or molded pieces of such melamine-formaldehyde foam, and in particular melamine foam, have become popular to remove soils and/or stains from hard surfaces (i.e., cleaning of hard surfaces) such as tiles, walls, floors, sanitary fittings such as sinks, showers, shower curtains, wash basins, WCs, household appliances including, but not limited to, refrigerators, freezers, washing machines, automatic dryers, ovens, microwave ovens, dishwashers and so on. Melamine foam sponges are currently marketed under the tradename Mr. Clean Magic Eraser^{®}.

### SUMMARY

It has been observed that melamine-formaldehyde foams show good soil and/or stain removal performance when used to clean hard surfaces, or to remove stains and/or soils such as marks on walls and furniture. Indeed, it has been observed that open-cell melamine foam, when wetted with an appropriate solvent, such as tap water, removes soils and/or stains from a hard surface when said hard surface is brought into contact with said wetted modified melamine foam. By "bringing into contact" it is meant wiping, swiping, rubbing or the like. In order for the melamine foam to optimally remove soils and/or stains from hard surfaces substantial amounts of an appropriate solvent, such as tap water, have to be used. Most commonly, tap water is used by the users of melamine foam when removing soils and/or stains from hard surfaces. When used with water or any other appropriate solvent, the melamine foam can come off as small particles (meaning, the foam crumbles) when brought into contact with a hard surface. Indeed, a milky suspension of small, melamine foam particles in water is formed. There is therefore the need for better soil and/or stain removal with better durability of melamine foams upon use.

The present disclosure addresses this need by providing a cleaning implement having an increased thickness, wherein said implement is capable of improved cleaning from hard surfaces while showing excellent durability upon use. Attributes as described in this disclosure produce an implement that holds up for longer while cleaning and better utilizes the force applied by distributing that force over a wider area, allowing cleaning to occur with less manual effort.

According to the subject matter of the present disclosure, an embodiment includes a cleaning implement that includes a foam having a density from 8 to 36 kg/m³. The cleaning implement has a thickness, a width, and a depth. The thickness is at least 22 mm, the width is less than or substantially equal to the depth, and the width is greater than the thickness.

In accordance with another embodiment of the present disclosure, a method of cleaning a hard surface includes bringing the cleaning implement into contact with a hard surface.

### BRIEF DESCRIPTION OF THE DRAWINGS:

The following detailed description of specific embodiments of the present disclosure can be best understood when read in conjunction with the following drawings, where like structure is indicated with like reference numerals and in which:
FIG. 1A is a perspective view of a cleaning implement, according to embodiments of the present disclosure;
FIG. 1B is a perspective view of a cleaning implement, according to embodiments of the present disclosure;
FIG. 2 is a perspective view of a cleaning implement, according to embodiments of the present disclosure; and
FIG. 3 is a graph of force distribution area over total force applied for cleaning implements, according to embodiments of the present disclosure.

### DETAILED DESCRIPTION

As used herein, the term "cleaning implement" refers to an article of manufacture of any suitable shape and/or size and/or volume suitable for cleaning, i.e., removing spots and/or stains from hard surfaces. The cleaning implement herein has a shape and/or size and/or volume suitable for use by a consumer to clean hard surfaces therewith. Examples of cleaning implements are wipers, brushes, cleaning cloths or cleaning granules.

As used herein, the term "household hard surface," it is meant herein any kind of surface typically found in and around houses like kitchens, bathrooms, e.g., floors, walls, tiles, windows, cupboards, sinks, showers, shower plastified curtains, wash basins, WCs, fixtures and fittings and the like made of different materials like ceramic, vinyl, no-wax vinyl, linoleum, melamine, glass, Inox^{®}, Formica^{®}, any plastics, plastified wood, metal or any painted or varnished or sealed surface and the like. Household hard surfaces also include household appliances including, but not limited to refrigerators, freezers, washing machines, automatic dryers, ovens, microwave ovens, dishwashers and so on. Such hard surfaces may be found both in private households as well as in commercial, institutional and industrial environments.

The disclosures of US Patent 6,608,118, US Patent 9,174,247, US Patent 11,224,328, US Patent 11,259,680, US Patent 8,283,305, US Patent 8,206,820, US Patent 8,277,935, US Patent 10,292,561 are hereby incorporated by reference in their entirety.

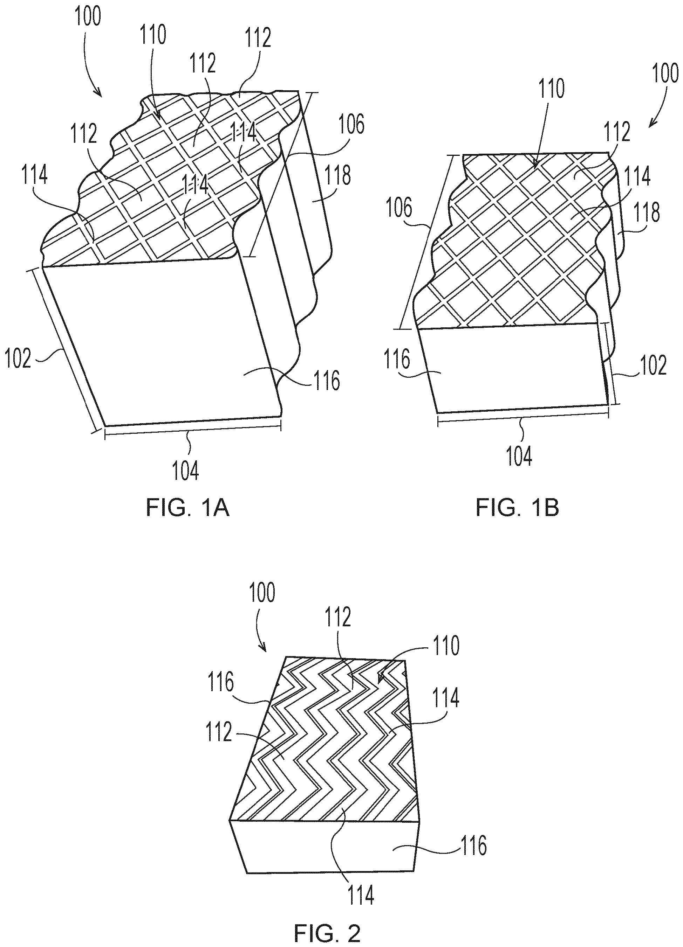

Referring initially to FIG. 1, the present disclosure relates to a cleaning implement 100 having a thickness 102, a width 104, and a depth 106. The cleaning implement includes a foam having a density from 8 to 36 kg/m³. The cleaning implements 100 of the present disclosure are suitable for cleaning/cleansing inanimate surfaces such as household hard surfaces, dish surfaces, leather, synthetic leather, the surfaces of automotive vehicles, or combinations thereof.

As stated previously, the foam has a density from 8 to 36 kilograms per cubic meter (kg/m³), from 8 to 35 kg/m³, from 8 to 30 kg/m³, from 8 to 25 kg/m³, from 8 to 20 kg/m³, from 8 to 15 kg/m³, from 8 to 10 kg/m³, from 10 to 36 kg/m³, from 10 to 35 kg/m³, from 10 to 30 kg/m³, from 10 to 25 kg/m³, from 10 to 20 kg/m³, from 10 to 15 kg/m³, from 15 to 36 kg/m³, from 15 to 35 kg/m³, from 15 to 30 kg/m³, from 15 to 25 kg/m³, from 15 to 20 kg/m³, from 17 to 36 kg/m³, from 17 to 35 kg/m³, from 17 to 30 kg/m³, from 17 to 25 kg/m³, from 17 to 20 kg/m³, from 20 to 36 kg/m³, from 20 to 35 kg/m³, from 20 to 30 kg/m³, from 20 to 25 kg/m³, from 25 to 36 kg/m³, from 25 to 35 kg/m³, from 25 to 30 kg/m³, from 30 to 36 kg/m³, from 30 to 35 kg/m³, or any values within the foregoing ranges or any ranges created thereby. It is contemplated that foam having a density within this range holds up better to tougher scrubbing, such as scrubbing highly textured surfaces and/or sticky/dried/burnt on soils. Additionally or alternatively, foam having a density within this range may be more durable so that when consumers scrub the tough surfaces less particles fall off the foam, it takes longer for the foam to deteriorate, or both. Foams having a lesser density than the provided ranges tend to fall apart faster, leaving significant particles behind for consumers to clean-up after they cleaned their soiled surfaces. Foams having a lesser density than the provided ranges also do not hold up to cleaning some of the toughest soils (dried/sticky soils), thus not allowing consumers to fully clean their surface. Foams having a greater density than the provided ranges tend to be more rigid, harder for consumers to hold and may be more brittle (falls apart or shreds more easily).

The cleaning implement 100 has a thickness 102 of at least 22 millimeters (mm), at least 25 mm, at least 28 mm, at least 30 mm, at least 33 mm, or at least 35 mm. In embodiments, the thickness may range from 22 to 45 mm, from 22 to 40 mm, from 22 to 35 mm, from 22 to 33 mm, from 22 to 30 mm, from 22 to 28 mm, from 22 to 25 mm, from 25 to 45 mm, from 25 to 40 mm, from 25 to 35 mm, from 25 to 33 mm, from 25 to 30 mm, from 25 to 28 mm, from 28 to 45 mm, from 28 to 40 mm, from 28 to 35 mm, from 28 to 33 mm, from 28 to 30 mm, from 30 to 45 mm, from 30 to 40 mm, from 30 to 35 mm, from 30 to 33 mm, from 33 to 45 mm, from 33 to 40 mm, from 33 to 35 mm, from 35 to 45 mm, from 35 to 40 mm, from 40 to 45 mm, or any values within the foregoing ranges or any ranges created thereby. It is contemplated that increasing the thickness of the cleaning implement may improve performance of the cleaning implement. FIG. 1 shows a comparison of two cleaning implements 100 having differing thicknesses 102. Improved performance may include an "easier scrubbing" feel to the consumer, resulting in less effort while cleaning. Specifically, a cleaning implement 100 having a thickness 102 of at least 22 mm, at least 25 mm, at least 28 mm, at least 30 mm, or any of the thicknesses 102 previously described may exhibit decreased consumer effort/scrubbing effort as compared to the consumer effort/scrubbing effort when a cleaning implement 100 having a lesser thickness is used. It is contemplated that the increased thickness 102 results in a "built-in handle" present on the cleaning implement 100, as compared to cleaning implements 100 with lesser thickness 102, and that this increased thickness 102, or "built-in handle", is directly related to consumer ease-of-use and scrubbing efficiency. Additionally or alternatively, a cleaning implement 102 having a relatively greater thickness (i.e. a thickness 102 within the above-provided ranges) provides improved ergonomics while cleaning, due to the built-in-handle/thickness. The force applied to the top surface or bottom surface of the cleaning implement 100 by a consumer when scrubbing results in a larger surface area of the top surface or the bottom surface of the cleaning implement 100 in contact with the cleaning surface. This allows for cleaning a larger amount of soil at a time with seemingly less effort than with thinner cleaning implements 100 where pressure remains more localized. Cleaning implements 100 having a lesser thickness than the ranges provided above are harder for a consumer to hold and use. The thinner cleaning implements 100 'flop' out of consumers' hands easier while scrubbing. Consumers tend to 'fold' thinner cleaning implements 100 in half to get a better grip on the eraser to scrub with. Consumers also tend to need to use both sides (top surface and bottom surface) of the cleaning implement 100 when cleaning with thinner cleaning implements 100 having a thickness 102 less than the provided ranges. Cleaning implements 100 having a thickness 102 greater than the provided ranges generally are too hard for most consumers to hold onto. While the thicker foam provides a handle, it is too hard to hold to scrub effectively with.

As stated previously, the cleaning implement 100 has a width 104 and a depth 106. The width 104 is greater than the thickness 102. Additionally, the width 104 of the cleaning implement 100 is less than or substantially equal to the depth 106. In embodiments, the width 104 is less than the depth 106.

In embodiments, the width 104 of the cleaning implement 100 may range from 46 to 125 mm, from 46 to 120 mm, from 46 to 110 mm, from 46 to 100 mm, from 46 to 90 mm, from 46 to 80 mm, from 46 to 75 mm, from 46 to 70 mm, from 46 to 60 mm, from 46 to 50 mm, from 50 to 125 mm, from 50 to 120 mm, from 50 to 110 mm, from 50 to 100 mm, from 50 to 90 mm, from 50 to 80 mm, from 50 to 75 mm, from 50 to 70 mm, from 50 to 60 mm, from 60 to 125 mm, from 60 to 120 mm, from 60 to 110 mm, from 60 to 100 mm, from 60 to 90 mm, from 60 to 80 mm, from 60 to 75 mm, from 60 to 70 mm, from 70 to 125 mm, from 70 to 120 mm, from 70 to 110 mm, from 70 to 100 mm, from 70 to 90 mm, from 70 to 80 mm, from 70 to 75 mm, from 75 to 125 mm, from 75 to 120 mm, from 75 to 110 mm, from 75 to 100 mm, from 75 to 90 mm, from 75 to 80 mm, from 80 to 125 mm, from 80 to 120 mm, from 80 to 110 mm, from 80 to 100 mm, from 80 to 90 mm, from 90 to 125 mm, from 90 to 120 mm, from 90 to 110 mm, from 90 to 100 mm, from 100 to 125 mm, from 100 to 120 mm, from 100 to 110 mm, from 110 to 125 mm, from 110 to 120 mm, from 120 to 125 mm, or any values within the foregoing ranges or any ranges created thereby. If the cleaning implement 100 is wider than the provided width 104 ranges, then it is too hard for consumers to effectively grip and hold the cleaning implement 100 to scrub with. If the cleaning implement 100 is thinner than the provided width 104 ranges, it becomes awkward for consumers to effectively grip and scrub with.

In embodiments, the depth 106 of the cleaning implement 100 may range from 46 to 125 mm, from 46 to 120 mm, from 46 to 110 mm, from 46 to 100 mm, from 46 to 90 mm, from 46 to 80 mm, from 46 to 75 mm, from 46 to 70 mm, from 46 to 60 mm, from 46 to 50 mm, from 50 to 125 mm, from 50 to 120 mm, from 50 to 110 mm, from 50 to 100 mm, from 50 to 90 mm, from 50 to 80 mm, from 50 to 75 mm, from 50 to 70 mm, from 50 to 60 mm, from 60 to 125 mm, from 60 to 120 mm, from 60 to 110 mm, from 60 to 100 mm, from 60 to 90 mm, from 60 to 80 mm, from 60 to 75 mm, from 60 to 70 mm, from 70 to 125 mm, from 70 to 120 mm, from 70 to 110 mm, from 70 to 100 mm, from 70 to 90 mm, from 70 to 80 mm, from 70 to 75 mm, from 75 to 125 mm, from 75 to 120 mm, from 75 to 110 mm, from 75 to 100 mm, from 75 to 90 mm, from 75 to 80 mm, from 80 to 125 mm, from 80 to 120 mm, from 80 to 110 mm, from 80 to 100 mm, from 80 to 90 mm, from 90 to 125 mm, from 90 to 120 mm, from 90 to 110 mm, from 90 to 100 mm, from 100 to 125 mm, from 100 to 120 mm, from 100 to 110 mm, from 110 to 125 mm, from 110 to 120 mm, from 120 to 125 mm, or any values within the foregoing ranges or any ranges created thereby. If the cleaning implement 100 has a depth 106 greater than the provide ranges, it becomes floppy and awkward to hold to enable effective cleaning (poor ergonomics). If the cleaning implement 100 has a depth 106 less than the provide ranges, it is harder to grip to scrub effectively with.

In embodiments, the cleaning implement 100 may have a shape including cube shape (not shown), rectangular prism shape (as shown in FIG. 2), pyramid shape (not shown), cylindrical shape (not shown), cone shape (not shown), pencil eraser shape (not shown), cuboid shape (not shown), tetrahedron shape (not shown), sphere shape (not shown), globular shape (not shown), ellipsoid shape (not shown), or combinations thereof. In embodiments, the cleaning implement 100 may have a substantially rectangular prism shape having two curved sides 118 opposite each other and two substantially smooth and/or straight sides 116 opposite each other, as shown in FIG. 1. In embodiments, the wave pattern may resemble a sinusoidal wave, meaning a curve with a smooth repetitive oscillation, as shown in FIG. 1.

In embodiments, the cleaning implement 100 has a cross-sectional surface area ranging from 1012 to 5625 square millimeters (mm²). As used herein, the term "cross-sectional surface area" refers to the cross-section of the cleaning implement 100 defined by the thickness 102 and width 104. In embodiments, the cross-sectional surface area may range from 1012 to 5625 mm², from 1012 to 5500 mm², from 1012 to 5000 mm², from 1012 to 4500 mm², from 1012 to 4000 mm², from 1012 to 3500 mm², from 1012 to 3000 mm², from 1012 to 2500 mm², from 1012 to 2000 mm², from 1012 to 1500 mm², from 1500 to 5625 mm², from 1500 to 5500 mm², from 1500 to 5000 mm², from 1500 to 4500 mm², from 1500 to 4000 mm², from 1500 to 3500 mm², from 1500 to 3000 mm², from 1500 to 2500 mm², from 1500 to 2000 mm², from 2000 to 5625 mm², from 2000 to 5500 mm², from 2000 to 5000 mm², from 2000 to 4500 mm², from 2000 to 4000 mm², from 2000 to 3500 mm², from 2000 to 3000 mm², from 2000 to 2500 mm², from 2500 to 5625 mm², from 2500 to 5500 mm², from 2500 to 5000 mm², from 2500 to 4500 mm², from 2500 to 4000 mm², from 2500 to 3500 mm², from 2500 to 3000 mm², from 3000 to 5625 mm², from 3000 to 5500 mm², from 3000 to 5000 mm², from 3000 to 4500 mm², from 3000 to 4000 mm², from 3000 to 3500 mm², from 3500 to 5625 mm², from 3500 to 5500 mm², from 3500 to 5000 mm², from 3500 to 4500 mm², from 3500 to 4000 mm², from 4000 to 5625 mm², from 4000 to 5500 mm², from 4000 to 5000 mm², from 4000 to 4500 mm², from 4500 to 5625 mm², from 4500 to 5500 mm², from 4500 to 5000 mm², from 5000 to 5625 mm², from 5000 to 5500 mm², or any values within the foregoing ranges or any ranges created thereby.

The cleaning implement 100 may have a volume from 1 to 10,000 cubic centimeters (cc). In embodiments, the cleaning implement 100 may have a volume from 40 to 750 cc, from 40 to 700 cc, from 40 to 600 cc, from 40 to 500 cc, from 40 to 400 cc, from 40 to 300 cc, from 40 to 200 cc, from 40 to 100 cc, from 40 to 50 cc, from 50 to 750 cc, from 50 to 700 cc, from 50 to 600 cc, from 50 to 500 cc, from 50 to 400 cc, from 50 to 300 cc, from 50 to 200 cc, from 50 to 100 cc, from 100 to 750 cc, from 100 to 700 cc, from 100 to 600 cc, from 100 to 500 cc, from 100 to 400 cc, from 100 to 300 cc, from 100 to 200 cc, from 200 to 750 cc, from 200 to 700 cc, from 200 to 600 cc, from 200 to 500 cc, from 200 to 400 cc, from 200 to 300 cc, from 300 to 750 cc, from 300 to 700 cc, from 300 to 600 cc, from 300 to 500 cc, from 300 to 400 cc, from 400 to 750 cc, from 400 to 700 cc, from 400 to 600 cc, from 400 to 500 cc, from 500 to 750 cc, from 500 to 700 cc, from 500 to 600 cc, from 600 to 750 cc, from 600 to 700 cc, from 700 to 750 cc, or any values within the foregoing ranges or any ranges created thereby.

The cleaning implement 100 may have an average pore diameter from 1 to 1000 microns (µm). As used throughout this disclosure, the term "average pore diameter" refers to the average or effective diameter of the openings in a porous material, such as the foam of the cleaning implement. In embodiments, the cleaning implement 100 may have an average pore diameter from 1 to 1000 µm, from 1 to 900 µm, from 1 to 800 µm, from 1 to 700 µm, from 1 to 600 µm, from 1 to 500 µm, from 1 to 400 µm, from 1 to 300 µm, from 1 to 200 µm, from 1 to 100 µm, from 100 to 1000 µm, from 100 to 900 µm, from 100 to 800 µm, from 100 to 700 µm, from 100 to 600 µm, from 100 to 500 µm, from 100 to 400 µm, from 100 to 300 µm, from 100 to 200 µm, from 200 to 1000 µm, from 200 to 900 µm, from 200 to 800 µm, from 200 to 700 µm, from 200 to 600 µm, from 200 to 500 µm, from 200 to 400 µm, from 200 to 300 µm, from 300 to 1000 µm, from 300 to 900 µm, from 300 to 800 µm, from 300 to 700 µm, from 300 to 600 µm, from 300 to 500 µm, from 300 to 400 µm, from 400 to 1000 µm, from 400 to 900 µm, from 400 to 800 µm, from 400 to 700 µm, from 400 to 600 µm, from 400 to 500 µm, from 500 to 1000 µm, from 500 to 900 µm, from 500 to 800 µm, from 500 to 700 µm, from 500 to 600 µm, from 600 to 1000 µm, from 600 to 900 µm, from 600 to 800 µm, from 600 to 700 µm, from 700 to 1000 µm, from 700 to 900 µm, from 700 to 800 µm, from 800 to 1000 µm, from 800 to 900 µm, from 900 to 1000 µm, or any values within the foregoing ranges or any ranges created thereby.

In embodiments, the cleaning implement 100 may exhibit a dry tensile strength of from 40 to 105 Newtons (N), from 40 to 100 N, from 40 to 95 N, from 40 to 93 N, from 40 to 90 N, from 50 to 105 N, from 50 to 100 N, from 50 to 95 N, from 50 to 93 N, from 50 to 90 N, from 60 to 105 N, from 60 to 100 N, from 60 to 95 N, from 60 to 93 N, from 60 to 90 N, from 70 to 105 N, from 70 to 100 N, from 70 to 95 N, from 70 to 93 N, from 70 to 90 N, from 73 to 105 N, from 73 to 100 N, from 73 to 95 N, from 73 to 93 N, from 73 to 90 N, from 80 to 105 N, from 80 to 100 N, from 80 to 95 N, from 80 to 93 N, from 80 to 90 N, or any values within the foregoing ranges or any ranges created thereby.

In embodiments, the cleaning implement 100 may exhibit a wet tensile strength of from 80 to 150 N, from 80 to 140 N, from 80 to 135 N, from 80 to 130 N, 85 to 150 N, from 85 to 140 N, from 85 to 135 N, from 85 to 130 N, 90 to 150 N, from 90 to 140 N, from 90 to 135 N, from 90 to 130 N, from 95 to 150 N, from 95 to 140 N, from 95 to 135 N, from 95 to 130 N, from 100 to 150 N, from 100 to 140 N, from 100 to 135 N, from 100 to 130 N, from 105 to 150 N, from 105 to 140 N, from 105 to 135 N, from 105 to 130 N, from 110 to 150 N, from 110 to 140 N, from 110 to 135 N, from 110 to 130 N, or any values within the foregoing ranges or any ranges created thereby.

In embodiments, the cleaning implement 100 may exhibit a force distribution area of greater than 5 square inches (in²), greater than 6 in², greater than 6.5 in², greater than 7 in², greater than 7.5 in², greater than 8 in², greater than 8.5 in², greater than 9 in², greater than 9.5 in², greater than 10 in², greater than 10.5 in², greater than 11 in², or greater than 11.5 in² when a total force of greater than or equal to about 0.5 pounds (lbs), greater than or equal to 0.75 lbs, greater than 1 lbs, greater than 1.5 lbs, greater than 2 lbs, greater than 2.5 lbs, greater than 3 lbs, greater than 3.5 lbs, or greater than 4 lbs is applied. In embodiments, the cleaning implement 100 may exhibit a force distribution area of from 5 to 15 in², from 5 to 14 in², from 5 to 13 in², from 5 to 12 in², from 5 to 11 in², from 5 to 10 in², from 5 to 9 in², from 5 to 8 in², from 5 to 7.5 in², from 5 to 7 in², or from 5 to 6 in², from 6 to 15 in², from 6 to 14 in², from 6 to 13 in², from 6 to 12 in², from 6 to 11 in², from 6 to 10 in², from 6 to 9 in², from 6 to 8 in², from 6 to 7.5 in², from 6 to 7 in², from 7 to 15 in², from 7 to 14 in², from 7 to 13 in², from 7 to 12 in², from 7 to 11 in², from 7 to 10 in², from 7 to 9 in², from 7 to 8 in², from 7 to 7.5 in², from 7.5 to 15 in², from 7.5 to 14 in², from 7.5 to 13 in², from 7.5 to 12 in², from 7.5 to 11 in², from 7.5 to 10 in², from 7.5 to 9 in², from 7.5 to 8 in², from 8 to 15 in², from 8 to 14 in², from 8 to 13 in², from 8 to 12 in², from 8 to 11 in², from 8 to 10 in², from 8 to 9 in², from 9 to 15 in², from 9 to 14 in², from 9 to 13 in², from 9 to 12 in², from 9 to 11 in², from 9 to 10 in², from 10 to 15 in², from 10 to 14 in², from 10 to 13 in², from 10 to 12 in², from 10 to 11 in², from 11 to 15 in², from 11 to 14 in², from 11 to 13 in², from 11 to 12 in², from 10.5 to 11.5 in² or any values within the foregoing ranges or any ranges created thereby when a total force of from 0.5 to 6.5 lbs, from 0.5 to 6 lbs, from 0.5 to 5 lbs, from 0.5 to 4 lbs, from 0.5 to 3.5 lbs, from 0.5 to 3 lbs, from 0.5 to 2.5 lbs, from 0.5 to 2 lbs, from 0.5 to 1.5 lbs, from 0.5 to 1 lbs, from 0.5 to 0.75 lbs, from 0.75 to 6.5 lbs, from 0.75 to 6 lbs, from 0.75 to 5 lbs, from 0.75 to 4 lbs, from 0.75 to 3.5 lbs, from 0.75 to 3 lbs, from 0.75 to 2.5 lbs, from 0.75 to 2 lbs, from 0.75 to 1.5 lbs, from 0.75 to 1 lbs, from 1 to 6.5 lbs, from 1 to 6 lbs, from 1 to 5 lbs, from 1 to 4 lbs, from 1 to 3.5 lbs, from 1 to 3 lbs, from 1 to 2.5 lbs, from 1 to 2 lbs, from 1 to 1.5 lbs, from 1.5 to 6.5 lbs, from 1.5 to 6 lbs, from 1.5 to 5 lbs, from 1.5 to 4 lbs, from 1.5 to 3.5 lbs, from 1.5 to 3 lbs, from 1.5 to 2.5 lbs, from 1.5 to 2 lbs, from 2 to 6.5 lbs, from 2 to 6 lbs, from 2 to 5 lbs, from 2 to 4 lbs, from 2 to 3.5 lbs, from 2 to 3 lbs, from 2 to 2.5 lbs, from 2.5 to 6 lbs, from 2.5 to 5 lbs, from 2.5 to 4 lbs, from 2.5 to 3.5 lbs, from 2.5 to 3 lbs, from 3 to 6 lbs, from 3 to 5 lbs, from 3 to 4 lbs, from 3 to 3.5 lbs, from 3.5 to 6 lbs, from 3.5 to 5 lbs, from 3.5 to 4 lbs, from 4 to 6 lbs, from 4 to 5 lbs, from 5 to 6 lbs or any values within the foregoing ranges or any ranges created thereby is applied.

As stated previously, the cleaning implement 100 includes foam. In embodiments, the foam may include melamine. In embodiments, the foam includes melamine-formaldehyde. Melamine-formaldehyde foams as such and their production are known to a person skilled in the art and described in the literature.

In order to obtain suitable cleaning implements 100 the foam may have to be modified in shape and/or size. This modification can be done by any means known to those skilled in the art. Suitable means of modifying the shape and/or size of foam raw materials may include cutting, breaking, tearing, or combinations thereof.

In embodiments where the cleaning implement 100 is modified in shape or size, the foam may be subjected to compression molding. The foam may originally be either a slab foam obtained in an open mold or a mold foam obtained in a closed mold. The melamine molded foam may be formed by compressing the melamine foam to a thickness of 1/1.2 to 1/12 as in the second aspect of the invention. That is, the ratio of the thickness of the melamine foam after heat-compression to the thickness of the melamine foam before heat compression (which is hereinafter referred to as "degree of compression") is from 1/1.2 to 1/12. In case when the degree of compression is too small, there might result a molded foam with an insufficient strength and an insufficiently reduced fragility. On the other hand, in case when the melamine foam is compressed to a thickness as thin as less than 1/12, i.e., in case when the degree of compression is too large, there tends to result an insufficient resistance to moist heat aging. The molded foam is formed by compressing the melamine foam to a thickness of preferably 1/1.5 to 1/10, more preferably 1/2 to 1/7. Compression to a thickness of this range can provide a melamine molded foam having a sufficiently reduced fragility and an excellent resistance to moist heat aging. The melamine molded foam can be formed by plastically deforming a melamine foam. When the melamine foam can be completely plastically deformed by heat-compression, its shape and dimension will not greatly recover, as in it will not return to the original dimensions. Furthermore, such a molded foam has been actually obtained. However, the dimensional change in the direction of thickness with the lapse of time is observed depending upon the heat compression conditions.

The cleaning implement 100 may have a top surface 110. In embodiments, the top surface 110 may be flat (not shown), or may have a textured surface (shown in FIGS. 1 and 2) which may result in excellent rub-cleaning properties. This may be attributed to that the textured surface of the top surface 110 of the foam catches the fine unevenness formed by the dirt existing on the stained surface and exerts an action of strongly scratching the dirt on the stained surface. The textured surface may include raised portions 112 and depressed portions 114. The raised portions 112 and depressed portions 114 may form patterns on the top surface 110, as shown in FIGS. 1 and 2. In embodiments, the pattern may include where the raised portions 112 form diamond shapes between the depressed portions 114 where the depressed portions 114 form criss-crossing diagonal lines, as shown in FIG. 1. In embodiments, the pattern may include where the raised portions 112 and the depressed portions 114 form chevrons as shown in FIG. 2.

The cleaning implements described herein may include a benefit agent, located anywhere as known in the art. In embodiments, the active agent is impregnated in the foam. The benefit agents may include biocides, bleaching agents, limescale reducing agents, solvents, polymers, solids, surfactants, colorants, lubricants, cross-linkers, fragrances, plasticizers, odor scavengers, microcapsules, or combinations thereof. Colorants may include dyes, pigments, or both.

In embodiments, the benefit agent may have an HLB greater than 5, greater than 8, greater than 10, or greater than 12. In embodiments, the HLB of the benefit agent may range from 5 to 14, from 5 to 12, from 5 to 10, from 5 to 8, from 8 to 14, from 8 to 12, from 8 to 10, from 10 to 14, from 10 to 12, from 12 to 14, or any values within the foregoing ranges or any ranges created thereby.

The benefit agent may be present in free form in an amount from about 5% to about 20%, or from about 10% to about 15% by weight of the cleaning implement. A benefit agent in free form means that the benefit agent is supplied to the cleaning implement in its neat form whose release from the cleaning implement is not purposefully controlled, delayed, or sustained. In some preferred embodiments, the benefit agent(s) is supplied to the cleaning implement in forms, as known in the art, such that the release from the cleaning implement is purposefully controlled, delayed, or sustained.

The benefit agent may include a surfactant. The surfactant may include nonionic, anionic, cationic, amphoteric, or zwitterionic surfactants. Suitable nonionic surfactants include alkoxylated fatty alcohol having the formula of RO(EO)e(PO)pH, where R is a hydrocarbon chain of from 2 to 24 carbon atoms, EO is ethylene oxide and PO is propylene oxide, e and p respectively representing the average degree of ethoxylation and propoxylation, are independently from 0 to 24, or R is a straight alkyl chain having from 6 to 22 carbon atoms, e is 5-12 and p is 0 (e.g. Lutensol^{™}). Suitable cationic surfactants herein include derivatives of quaternary ammonium, phosphonium, imidazolium and sulfonium compounds. Preferred cationic surfactants herein are trimethyl quaternary ammonium compounds. Suitable amphoteric surfactants herein include amine oxides, betaine or ammonium sulfate or ammonium carboxylate, having the following formula R₁R₂R₃NO, R₁R₂R₃NR₄SO₄ or R₁R₂R₃NR₄CO₂ wherein each of R₁, R₂ and R₃ is independently a saturated substituted or unsubstituted, linear or branched alkyl groups of from 1 to 30, or from 8 to 18 carbon atoms, except for R₄ which preferably include 3 saturated carbons. Preferred amine oxides herein are for instance natural blend C₈-C₁₀ amine oxides, and C₁₂-C₁₆ amine oxides, such as cetyl dimethyl amine oxide. Preferred betaine herein is cocamidopropyl betaine and lauramidopropyl betaine. Suitable anionic surfactants include alkyl diphenyl ether sulphonate and alkyl carboxylate. Other suitable anionic surfactants herein include water soluble salts or acids of the formula ROSO₃M wherein R is preferably a C₁₀-C₂₄ hydrocarbyl, or C₁₂-C₁₈ alkyl or hydroxyalkyl, and M is H or a cation, such as sodium, potassium, lithium, or ammonium or substituted ammonium. Other suitable anionic surfactants include soap salts, C₉-C₂₀ linear alkylbenzenesulfonates, C₈-C₂₂ primary or secondary alkylsulfonates, sulfonated polycarboxylic acids, C₈-C₂₄ alkylpolyglycolethersulfates (includeing up to 10 moles of ethylene oxide); alkyl ester sulfonates, sulfates of alkyl polysaccharides, alkyl polyethoxy carboxylates, such as those of the formula RO(CH₂CH₂O)ₖCH₂COO⁻M⁺ wherein R is a C₈-C₂₂ alkyl, k is an integer from 0 to 10, and M is a soluble salt-forming cation. Resin acids and hydrogenated resin acids are also suitable. Further examples are given in "Surface Active Agents and Detergents" (Vol. I and II by Schwartz, Perry and Berch). A variety of such surfactants are also generally disclosed in U.S. Patent 3,929,678.

Bleaching agents herein may be selected from a hydrogen peroxide source, a preformed peroxycarboxylic acid, a hypohalite bleach source, and a mixture thereof. Hydrogen peroxide sources herein include persulfate, dipersulphate, persulfuric acid, percarbonate, perborate, metal peroxide, perphosphate, persilicate, urea peroxyhydrate and a mixture thereof. Preformed peroxycarboxylic acids herein include those includeing one, two or more peroxy groups, and can be aliphatic or aromatic. When the organic percarboxylic acid is aliphatic, the unsubstituted acid suitably has the linear formula: HO-O-C(O)-(CH₂)ₙ-Y, wherein Y is H, CH₃, CH₂Cl, COOH or C(O)OOH; n is an integer of 1-20. Branched analogs are also acceptable. When the organic percarboxylic acid is aromatic, the unsubstituted acid suitably has formula: HO-O-C(O)-C₆H₄-Y wherein Y is hydrogen, alkyl, alkyhalogen, halogen, -COOH or -C(O)OOH. Monoperoxycarboxylic acids useful as oxygen bleach herein are further illustrated by alkyl percarboxylic acids and aryl percarboxylic acids such as peroxybenzoic acid and ring-substituted peroxybenzoic acids, e.g., peroxy-α-naphthoic acid; aliphatic, substituted aliphatic and arylalkyl monoperoxy acids such as peroxylauric acid, peroxystearic acid, and N,N-phthaloylaminoperoxycaproic acid (PAP); and 6-octylamino-6-oxo-peroxyhexanoic acid. Peracids can be used in acid form or any suitable salt with a bleach-stable cation. Suitable hypohalite bleaching agents herein include those that form positive halide ions and/or hypohalite ions, and bleaching agents that are organic based sources of halides, such as chloroisocyanurates. Suitable hypohalite bleaching agents herein include alkali metal and alkaline earth metal hypochlorite, hypobromite, hypoiodite, chlorinated trisodium phosphate dodecahydrate, potassium and sodium dichloroisocyanurates, potassium and sodium trichlorocyanurates, N-chloroimides, N-chloroamides, N-chloroamines and chlorohydantoins.

Limescale reducing agents herein include, but are not limited to, acids and chelating agents. Exemplary acids useful herein include hydrochloric acid, phosphoric acid, sulfuric acid, sulfamic acid, acetic acid, hydroxyacetic acid, citric acid, benzoic acid, tartaric acid, formic acid and mixtures thereof. A mixture of organic and inorganic acid is preferred. Chelating agents useful herein can include, but are not limited to, carboxylates, phosphates, phosphonates, polyfunctionally-substituted aromatic compounds, polyamines, biodegradable compounds, the alkali metal, ammonium or substituted ammonium salts or complexes of these chelating agents, and mixtures thereof. Further examples of suitable chelating agents and levels of use are described in U.S. Pat. Nos. 3,812,044; 4,704,233; 5,292,446; 5,445,747; 5,531,915; 5,545,352; 5,576,282; 5,641,739; 5,703,031; 5,705,464; 5,710,115; 5,710,115; 5,712,242; 5,721,205; 5,728,671; 5,747,440; 5,780,419; 5,879,409; 5,929,010; 5,929,018; 5,958,866; 5,965,514; 5,972,038; 6,172,021; and 6,503,876.

Biocide means any known ingredient having the ability of reducing or even eliminating by killing or removing the micro-organisms existing on a surface, such as those described in US 6,613,728. Biocide useful herein includes a quaternary surface active compound, a guanidine, an alcohol, a glycerol, a phenolic compound, a heavy metal salt, an inorganic and organic acid, a halogen, a halogen-includeing compound, a dye, an essential oil, an oxidizing compound, an adsorbent, a fungicide, an algaecide and a mixture thereof. Exemplary quaternary surface active compounds include benzalkonium chloride, benzethonium chloride, cetyl pyridinium chloride, sodium tetradecyl sulfate, sichlorobenzalkonium chloride, methylbenzethonium chloride, cetyl dimethyl ethyl ammonium bromide. Exemplary guanidines include chlorohexidine hydrochloride, chlorohexidine gluconate, dodecylguanidine hydrochloride, polyhexmethylenebiguanidine hydrochloride, and 6-acetoxy-2,4-dimethylmetadioxane. Exemplary alcohols include methanol, ethanol, propanol, isopropanol, etc. Exemplary phenolic compounds include cresol, resolcinols and related compounds, phenol; substituted phenols--cresols, meta-cresylacetate, creosote, quaiacol, resorcinol, hexylresorcinol, pyrogallol, thymol, thymol iodide, picric acid, chlorinated phenols--dichlorophene, hexachlorophene, tars. Exemplary halogens and halogen-includeing compounds include iodine and iodoform. Exemplary oxidizing agents include peroxide, sodium perporate, potassium permanganate, zinc permanganate, potassium chlorate. Exemplary heavy metal salts include mercuric chloride, miscellaneous ionizable mercuric salts, organic mercurials, silver nitrate, silver lactate, silver picrate, silver proteins, silver halides, zinc oxide, zinc stearate, copper sulfate and organic tin derivatives. Exemplary dyes include azo dyes, acridene dyes, fluorescein dyes, phenolphthalein dyes and triphenylmethane dyes. Exemplary inorganic and organic acids include hydrochloric acid, sulfuric acid, nitric acid, citric acid, sorbic acid, acetic acid, boric acid, formic acid, maleic acid, adipic acid, lactic acid, malic acid, malonic acid, glycolic acid, and mixtures thereof. Exemplary essential oils are thyme oil, clove oil, cinnamon oil, geranium oil, eucalyptus oil, peppermint oil, citronella oil, ajowan oil, mint oil or mixtures thereof. Other useful biocide herein includes furan derivatives, nitrofurantoin, sulfur, sulfur dioxide, ichthamol, chrysarobin, anthralin, betanaphthol, balsams, volatile oils, chlorophyl.

Biocides useful herein also include fungicides and algaecides which act against molds and mildew. Removal of algae and fungi from hard surfaces is difficult. Moreover, fungi and algae reappear promptly if not completely removed or inhibited. Suitable fungicides and algaecides include metal salts, such as zinc sulfate, zinc acetate, zinc bromide, zinc chloride, zinc iodide, zinc nitrate, zinc bromate and zinc chlorate, cooper halide, copper sulfate, organic tin derivatives, water-insoluble or partially water-soluble fungicides and algaecides, such as diiodomethyl p-tolyl sulfone, N-(trichloromethyl thio) phthalimide, N,N-dimethyl-N'-phenyl N'-(fluorodichloromethyl thio) sulphamide, 2-(thiocyanomethylthio) benzothiazole / methylene bis(thiocyanate), 3-iodo-2-propynyl butyl carbamate, etc., all available from ALDRICH chemical. Above biocides are optionally mixed with concentrated acids, such as acetic acid, formic, propionic, n-butanoic, n-pentanoic, trimethylacetic, n-hexanoic, lactic, methoxyacetic, cyanoacetic, chloroacetic, citric, partaric, etc.

The benefit agent may include a solvent having a good dissolving ability for greasy stains. Solvents useful herein include those which are at least partially water-miscible, such as alcohols, ethers, such as diethylene glycol diethylether, diethylene glycol dimethylether, propylene glycol dimethylether, propylene glycol monomethylether, propylene glycol monoethylether, propylene glycol monopropylether, propylene glycol monobutylether, ethylene glycol monobutylether, dipropylene glycol monomethylether, dipropylene glycol monopropyl ether, dipropylene glycol monobutyl ether, diethyleneglycol monobutylether, lower esters of monoalkylethers of ethylene glycol or propylene glycol, such as propylene glycol monomethyl ether acetate, N-methyl pyrolidone and tetrahydrofuran. Mixtures of several solvents may also be used.

Polymers useful herein include polyolefins, polyesters, polyvinyl chlorides, polyamides, mixtures thereof and copolymers thereof. Specific examples of useful polymers include but are not limited to polypropylene, polyethylene, polybutylene, polystyrene, polyethylene terephthalate, polyamide, polyacrylate, polyvinyl chloride, polyvinyl alcohol, ethylene vinyl acetate copolymers and mixtures thereof.

In embodiments, the cleaning implement may further include solids including abrasive materials, filler materials, or both. The solids may be inorganic or organic materials, e.g. sand, lime (CaCO₃), silicates with an average particle diameter (number-average) in the range from about 1 µm to about 1 mm, or colloidal silica, preferably inorganic material are selected from oxides, chlorides, sulfates, phosphates, carbonates of Mg, Mn, Ba, Ca, W, Zr, Ti, Si, Mo, in particular TiO₂, SiO₂, sand and Al₂O₃. Other suitable materials are insoluble sodium polymetaphosphate, hydrated alumina, dicalcium orthophosphate dihydrate, calcium pyrophosphate, tricalcium phosphate, calcium polymetaphosphate. Other abrasive material may be Carbon based materials : i.e.: as refered as black carbon, activated carbon, charcoal, etc. and may be porous or not. Other abrasive material include microspheres. Particularly preferred inorganic fillers are selected from zeolite based materials and silica based materials. Suitable zeolite based materials are described in the following reference texts: ZEOLITE SYNTHESIS, ACS Symposium Series 398, Eds. M. L. Occelli and H. E. Robson (1989) pages 2-7; ZEOLITE MOLECULAR SIEVES, Structure, Chemistry and Use, by D. W. Breck, John Wiley b Sons (1974) pages 245-250, 313-314 and 348-352 (all of which are incorporated herein by reference). SiO₂ exists in a variety of crystalline forms and amorphous modifications, any of which are suitable for use herein. In particular, silicas having a high surface area or in agglomerated forms are preferred (i.e., clays or shells). Without being restrictive to a family of silica based materials, commonly silica which is in a highly purified form such that is includes at least about 90%, preferably about 95%, more preferably about 99% silicon dioxide (i.e.: a silica gel having a about 100% silica content, and fumed silica) is preferred. Alternatively, silica based materials may be provided from other sources such as metal silicates including sodium silicate. Further suitable materials are water-insoluble sodium polymetaphosphate, hydrated alumina, dicalcium orthophosphate dihydrate, calcium pyrophosphate, tricalcium phosphate, calcium polymetaphosphate.

The cleaning implement described herein may be combined in an article of manufacture with a packaging means. The packaging means herein may be any suitable means known to package cleaning implements. Indeed, particularly suitable packaging means herein are selected from the group consisting of: paper bags, plastic bags, cartons, carton boxes, flow wraps, plastic wraps, and paper wraps, and the like and combinations thereof.

The packaging means herein may be printed and/or modified. In particular, such printing and/or other modification may be used to associate a brand-name and/or logo of a hard surface cleaner with said cleaning implement.

The present disclosure further relates to methods of cleaning a hard surface with the cleaning implement as described herein above.

The methods include bringing the cleaning implement of the present disclosure into contact with the hard surface. As used herein, the term "cleaning" refers to removing spots, stains, or both from hard surfaces.

Suitable hard surfaces herein are tiles, walls, floors, sanitary fittings such as sinks, showers, shower curtains, wash basins, WCs, household appliances including, but not limited to, refrigerators, freezers, washing machines, automatic dryers, ovens, microwave ovens, dishwashers and so on.

The methods may further include wetting said cleaning implement or said foam with an appropriate solvent, preferably tap water, more preferably water in combination with a detergent composition, prior to bringing said cleaning implement into contact with said hard surface.

### EXAMPLES

### Example 1: Consumer Testing

Consumer testing was conducted on various Examples of cleaning implements made according to the present disclosure. The results are shown in the Tables provided below, with consumer testing providing consumer perception values based out of 100.

**Table 1**

| Example | Example 1 | Example 2 | Example 3 | Example 4 | Example 5 | Example 6 |
|---|---|---|---|---|---|---|
| End Height / Thickness (mm) | 35 | 19.5 | 45 | 40 | 28 | 19.5 |
| Compression Ratio | 2.0 | 3.1 | 1.2 | 1.3 | 1.9 | 3.6 |
| Density (kg/m³) | 19.56 | 34.76 | 11.68 | 13.13 | 17.95 | 37.51 |
| length (cm) | 11.7 | 11.7 | 11.7 | 11.7 | 11.7 | 11.7 |
| width (cm) | 5.8 | 5.8 | 5.8 | 5.8 | 5.8 | 5.8 |
| height (cm) | 3.5 | 1.95 | 4.5 | 4 | 2.8 | 1.95 |
| weight (g) | 4.6 | 4.6 | 3.6 | 3.6 | 3.4 | 5.0 |
| | | | | | | |
| Overall Cleaning Ability | 80 | 57 | 65 | 66 | 77 | 63 |
| Satisfaction With Cleaning | 77 | 68 | 65 | 68 | 80 | 67 |
| Overall Durability | 64 | 43 | 46 | 59 | 68 | 48 |
| For The Amount Of Pilling Or Shredding When Using | 52 | 27 | 29 | 59 | 64 | 25 |
| For Lasting A Satisfactory Amount Of Time While Scrubbing Before The 1st Hole Appeared in the TEST PRODUCT | 73 | 48 | 42 | 61 | 68 | 48 |

**Table 2**

| Example | Example 7 | Example 8 | Example 9 | Comparative Example 1 (Walmart Extra Strength) | Example 10 | Example 11 |
|---|---|---|---|---|---|---|
| End Height / Thickness (mm) | 35 | 40 | 45 | 20.5 | 35 | 35 |
| Compression Ratio | 1.8 | 1.6 | 1.4 | n/a | 1.1 | 1.2 |
| Density (kg/m³) | 19.09 | 15.47 | 15.33 | 10.90 | 11.89 | 12.55 |
| length (cm) | 11.7 | 11.7 | 11.7 | 12.2 | 11.7 | 11.7 |
| width (cm) | 5.8 | 5.8 | 5.8 | 6.0 | 5.8 | 5.8 |
| height (cm) | 3.5 | 4 | 4.5 | 2.05 | 3.5 | 3.5 |
| weight (g) | 4.5 | 4.2 | 4.7 | 1.6 | 2.8 | 3.0 |
| | | | | | | |
| Overall Cleaning Ability | 56 | 59 | 68 | 52 | 69 | 55 |
| Satisfaction With Cleaning | 69 | 57 | 73 | 36 | 77 | 55 |
| Overall Durability | 50 | 50 | 55 | 11 | 58 | 34 |
| For The Amount Of Pilling Or Shredding When Using | 46 | 43 | 50 | 11 | 46 | 27 |
| For Lasting A Satisfactory Amount Of Time While Scrubbing Before The 1st Hole Appeared in the TEST PRODUCT | 67 | 52 | 64 | 11 | 63 | 41 |

**Table 3**

| Example | Example 12 | Example 13 | Example 14 | Example 15 | Example 16 | Comparativ e Example 2 (Walmart Original) |
|---|---|---|---|---|---|---|
| End Height / Thickness (mm) | 19.5 | 35 | 40 | 28 | 28 | 25 |
| Compression Ratio | 2 | 1.3 | 1.75 | 1.5 | 1.6 | n/a |
| Density (kg/m³) | 20.54 | 13.23 | 17.65 | 16.68 | 17.46 | 8.27 |
| length (cm) | 11.7 | 11.7 | 11.7 | 11.7 | 11.7 | 11.6 |
| width (cm) | 5.8 | 5.8 | 5.8 | 5.8 | 5.8 | 6.5 |
| height (cm) | 1.95 | 3.5 | 4 | 2.8 | 2.8 | 2.5 |
| weight (g) | 2.7 | 3.1 | 4.8 | 3.2 | 3.3 | 1.6 |
| | | | | | | |
| Overall Cleaning Ability | 36 | 83 | 73 | 75 | 58 | 40 |
| Satisfaction With Cleaning | 45 | 83 | 80 | 68 | 63 | 42 |
| Overall Durability | 30 | 71 | 66 | 59 | 48 | 21 |
| For The Amount Of Pilling Or Shredding When Using | 14 | 60 | 52 | 41 | 31 | 23 |
| For Lasting A Satisfactory Amount Of Time While Scrubbing Before The 1st Hole Appeared in the TEST PRODUCT | 27 | 79 | 75 | 59 | 40 | 29 |

**Table 4**

| Example | Example 17 | Example 18 | Example 19 | Example 20 | Example 21 | Example 22 |
|---|---|---|---|---|---|---|
| End Height / Thickness (mm) | 28 | 19.5 | 19.5 | 21 | 21 | 28 |
| Compression Ratio | 1.8 | 2.6 | 3 | 2.3 | 2.7 | 1 |
| Density (kg/m³) | 18.37 | 25.13 | 28.37 | 22.63 | 26.09 | 9.53 |
| length (cm) | 11.7 | 11.7 | 11.7 | 11.7 | 11.7 | 11.8 |
| width (cm) | 5.8 | 5.8 | 5.8 | 5.8 | 5.8 | 5.9 |
| height (cm) | 2.8 | 1.95 | 1.95 | 2.1 | 2.1 | 2.8 |
| weight (g) | 3.5 | 3.3 | 3.8 | 3.2 | 3.7 | 1.9 |
| | | | | | | |
| Overall Cleaning Ability | 70 | 71 | 50 | 83 | 77 | 65 |
| Satisfaction With Cleaning | 64 | 73 | 41 | 90 | 77 | 75 |
| Overall Durability | 52 | 54 | 43 | 69 | 59 | 40 |
| For The Amount Of Pilling Or Shredding When Using | 39 | 35 | 34 | 63 | 43 | 44 |
| For Lasting A Satisfactory Amount Of Time While Scrubbing Before The 1st Hole Appeared in the TEST PRODUCT | 57 | 56 | 57 | 73 | 66 | 46 |

**Table 5**

| Example | Example 23 | Example 24 | Example 25 | Example 26 |
|---|---|---|---|---|
| End Height / Thickness (mm) | 19.5 | 19.5 | 19.5 | 40 |
| Compression Ratio | 1.79 | 2 | 3.6 | 1.75 |
| Density (kg/m³) | 18.00 | 18.55 | 37.97 | 19.06 |
| length (cm) | 11.7 | 11.7 | 11.7 | 11.7 |
| width (cm) | 5.8 | 5.8 | 5.8 | 5.8 |
| height (cm) | 1.95 | 1.95 | 1.95 | 4 |
| weight (g) | 2.4 | 2.5 | 5.0 | 5.2 |
| | | | | |
| Overall Cleaning Ability | 45 | 77 | 69 | 66 |
| AU Satisfaction With Cleaning | 52 | 77 | 67 | 66 |
| Overall Durability | 30 | 55 | 65 | 52 |
| For The Amount Of Pilling Or Shredding When Using | 20 | 43 | 48 | 50 |
| For Lasting A Satisfactory Amount Of Time While Scrubbing Before The 1st Hole Appeared in the TEST PRODUCT | 34 | 66 | 67 | 57 |

### Examples 2-6

A number of commercially available "eraser" cleaning implements were purchased to compare to a cleaning implement made in accordance with the present disclosure. MCME Extra Durable (XD) and MCME Original are foam eraser cleaning implements available from Procter and Gamble, headquartered in Cincinnati, Ohio. Walmart Great Value Original and Walmart Great Value Extra Strength are foam eraser cleaning implements available from Walmart, headquartered in Bentonville, Arkansas. Target Up & Up Original and Target Up & Up Extra Strength are foam erasers cleaning implements available from Target, headquartered in Minneapolis, Minnesota. Kroger Cleaning Solutions is a foam eraser cleaning implement available from Kroger, headquartered in Cincinnati, Ohio.

### Example 2: Dimensions and Density

The dimensions and density of various cleaning implements were measured using a ruler marked with millimeters (mm) to measure the length, width, and height of the cleaning implement. The cleaning implements were then weighed on a Mettler Toledo Balance MS6002TS / C123929357. 3 cleaning implements with the target measurements/parameters of Example 27 were measured, 3 MCME XD foam erasers were measured, 3 MCME Original foam erasers were measured, 6 Walmart Great Value Original foam erasers were measured, 6 Walmart Great Value Extra Strength foam erasers were measured, 6 Target Up & Up Original foam erasers were measured, 6 Target Up & Up Extra Strength foam erasers were measured, and 6 Kroger Cleaning Solutions foam erasers were measured. The averages for each of the measurements for each product are shown below in Table 6.

**Table 6 -Average dimensions and density**

| Cleaning Implement | Length (mm) | Width (mm) | Height/Thickness (mm) | Weight (g) | Density (kg/m³) |
|---|---|---|---|---|---|
| Example 27 | 120 | 60 | 37 | 4.8 | 18 |
| MCME XD | 120 | 60 | 20 | 2.1 | 14 |
| MCME Original | 118 | 60 | 27 | 1.9 | 10 |
| Walmart Great Value Original | 117 | 61 | 25 | 2.0 | 11 |
| Walmart Great Value Extra Strength | 123 | 62 | 21 | 1.6 | 10 |
| Target Up & Up Original | 117 | 60 | 26 | 1.7 | 9 |
| Target Up & Up Extra Strength | 119 | 61 | 18 | 2.1 | 16 |
| Kroger Cleaning Solutions | 120 | 70 | 30 | 2.1 | 9 |

As is shown in Table 6, Example 27 has a greater average height/thickness than each of the comparative products. Additionally, Example 27 has a greater average density than each of the comparative products.

### Example 3: Dry Tensile Strength

The dry tensile strength of various cleaning implements were measured using an Electromechanical Load Frame available as MTS Criterion^{™} Model C41.103 from MTS System Corporation, headquartered in Minnesota, United States. The cleaning implement was placed between the grips of the electromechanical load frame and the grips were tightened and the cleaning implement was pulled by the electromechanical load frame until it was separated. The peak tensile force was then measured using the TW Elite software version 4.5.0.344 available from MTS TestSuite^{™} from MTS System Corporation.

**Table 7 - Individual measurements of dry tensile strength**

| Cleaning implement | Tensile Strength (N) |
|---|---|
| Great Value Original | 53 |
| Great Value Original | 49 |
| Great Value Original | 43 |
| Great Value Extra Strength | 61 |
| Great Value Extra Strength | 62 |
| Great Value Extra Strength | 60 |
| Target Up & Up Original | 49 |
| Target Up & Up Original | 46 |
| Target Up & Up Original | 45 |
| Target Up & Up Extra Strength | 63 |
| Target Up & Up Extra Strength | 60 |
| Target Up & Up Extra Strength | 60 |
| Kroger Cleaning Solutions | 37 |
| Kroger Cleaning Solutions | 51 |
| Kroger Cleaning Solutions | 76 |
| Example 27, Sample 1 | 102 |
| Example 27, Sample 2 | 74 |
| Example 27, Sample 3 | 85 |
| MCME XD | 94 |
| MCME XD | 95 |
| MCME XD | 93 |
| MCME Original | 53 |
| MCME Original | 56 |
| MCME Original | 63 |

**Table 8 - Average measurements of dry tensile strength**

| Cleaning implement | Tensile Strength (N) |
|---|---|
| Great Value Original | 48 |
| Great Value Extra Strength | 61 |
| Target Up & Up Original | 47 |
| Target Up & Up Extra Strength | 61 |
| Kroger Cleaning Solutions | 55 |
| Example 27 | 87 |
| MCME XD | 94 |
| MCME Original | 57 |

As is shown in Table 8, Example 27 has a greater average dry tensile strength than most of the comparative products. It is contemplated that the greater density and greater thickness provide an improved tensile strength as compared to most of the comparative products.

### Example 4: Wet Tensile Strength

The wet tensile strength of various cleaning implements was measured using an Electromechanical Load Frame available as MTS Criterion^{™} Model C41.103 from MTS System Corporation, headquartered in Minnesota, United States. The cleaning implement was first weighed dry. The cleaning implement was then wet with water and then all the excess water was squeezed out by hand. The cleaning implement was then weighed again to calculate the amount of water added. Then, the cleaning implement was placed between the grips of the electromechanical load frame and the grips were tightened and the cleaning implement was pulled by the electromechanical load frame until it was separated. The peak tensile force was then measured using the TW Elite software version 4.5.0.344 available from MTS TestSuite^{™} from MTS System Corporation.

**Table 9 - Individual measurements of wet tensile strength**

| Cleaning implement | Dry Weight (g) | Wet Weight (g) | Water Weight (g) | Tensile Strength (N) |
|---|---|---|---|---|
| Great Value Original | 2.02 | 29.89 | 27.87 | 56 |
| Great Value Original | 2.01 | 31.29 | 29.28 | 49 |
| Great Value Original | 1.92 | 33.09 | 31.17 | 50 |
| Great Value Extra Strength | 1.62 | 26.61 | 24.99 | 64 |
| Great Value Extra Strength | 1.65 | 25.45 | 23.8 | 63 |
| Great Value Extra Strength | 1.62 | 26.37 | 24.75 | 66 |
| Target Up & Up Original | 1.75 | 30.7 | 28.95 | 58 |
| Target Up & Up Original | 1.75 | 25.14 | 23.39 | 66 |
| Target Up & Up Original | 1.69 | 20.76 | 19.07 | 58 |
| Target Up & Up Extra Strength | 2.18 | 29.51 | 27.33 | 82 |
| Target Up & Up Extra Strength | 2.16 | 25.2 | 23.04 | 77 |
| Target Up & Up Extra Strength | 2.67 | 27.59 | 24.92 | 74 |
| Kroger Cleaning Solutions | 2.09 | 29.36 | 27.27 | 52 |
| Kroger Cleaning Solutions | 2.06 | 25.17 | 23.11 | 73 |
| Kroger Cleaning Solutions | 2.09 | 26.86 | 24.77 | 61 |
| Example 27, Sample 1 | 4.07 | 47.5 | 43.43 | 130 |
| Example 27, Sample 2 | 3.87 | 48.43 | 44.56 | 120 |
| Example 27, Sample 3 | 4.75 | 43.84 | 39.09 | 133 |
| MCME XD | 2.32 | 41.33 | 39.01 | 94 |
| MCME XD | 2.24 | 40.62 | 38.38 | 87 |
| MCME XD | 2.39 | 41.26 | 38.87 | 103 |
| MCME Original | 1.8 | 44.93 | 43.13 | 64 |
| MCME Original | 1.93 | 39.46 | 37.53 | 80 |
| MCME Original | 2.11 | 40.59 | 38.48 | 70 |

**Table 10 - Average measurements of wet tensile strength**

| Cleaning implement | Tensile Strength (N) |
|---|---|
| Great Value Original | 51.67 |
| Great Value Extra Strength | 64.33 |
| Target Up & Up Original | 60.67 |
| Target Up & Up Extra Strength | 77.67 |
| Kroger Cleaning Solutions | 62.00 |
| Example 27 | 127.67 |
| MCME XD | 94.67 |
| MCME Original | 71.33 |

As is shown in Table 10, Example 27 has a greater average wet tensile strength than all of the comparative products. It was surprising that the cleaning implement according to the present disclosure had a greater wet tensile strength than the comparative MCME XD, given that the MCME XD had a greater dry tensile strength than the Example 27. It is contemplated that the greater density and greater thickness provide an improved tensile strength as compared to the comparative products.

### Example 5: Durability Performance

The durability of the various cleaning implements was tested using scrubbing on a back of a tile and on a LEGO^{®} plate. For the tile, Daltile - Catalina Canyon Noce 12" x 12" Porcelain Floor & Wall Tile - Purchased from The Home Depot was used. For the LEGO^{®} plate, LEGO^{®} Classic White Baseplate 11026 from The LEGO^{®} Store was used. The procedure for the test is as follows. Fill 4L plastic beaker with lukewarm (80⁰F +/- 5⁰F) water. Place the large weigh boat on the analytical balance.

For the Back of Tile test, place a 12" x 14" piece of shelf liner on the lab bench. The shelf liner is used to hold the tile in place while scrubbing. It is helpful for the shelf liner to be larger than the tile itself. Place the Daltile Porcelain Tile on the shelf liner with the back of the tile facing upward. Place the dry eraser in a large weigh boat on the analytical balance and tare the balance. Then, submerge the melamine eraser into the plastic beaker with water and squeeze 5 times under water. This is to ensure the melamine eraser is fully wetted. Remove the melamine eraser and squeeze out excess water so the eraser is wet, but not dripping. Reweigh the eraser and record the weight. Keep the water weight consistent across each replicate. Start at the top center of the `back of the' porcelain tile. Hold the eraser in hand so that two fingers are pushing in the top middle of the eraser. While pushing down on the eraser, pull the eraser towards you until the eraser is about an inch from the end of the tile. This equals 1 stroke. Lift the eraser up and start back at the top of the tile for the next stroke. Repeat until you feel your finger touch the tile or you visually see the first hole appearing in the eraser. Record the number of strokes.

For the LEGO^{®} Plate test, place a 12" x 14" piece of shelf liner on the lab bench. The shelf liner is used to hold the LEGO^{®} plate in place while scrubbing. It is helpful for the shelf liner to be larger than the LEGO^{®} plate itself. Place the LEGO^{®} plate on the shelf liner facing upward. Place the dry eraser in a large weigh boat on the analytical balance and tare the balance. Submerge the melamine eraser into the plastic beaker with water and squeeze 5 times under water. This is to ensure the melamine eraser is fully wetted. Remove the melamine eraser and squeeze out excess water so the eraser is wet, but not dripping. Reweigh the eraser and record the weight. Keep the water weight consistent across each replicate. Start at the top center of the LEGO^{®} plate. Hold the eraser in hand so that two fingers are pushing in the top middle of the eraser. While pushing down on the eraser, pull the eraser towards you until the eraser is about an inch from the end of the LEGO^{®} plate. This equals 1 stroke. Lift the eraser up and start back at the top of the LEGO^{®} plate for the next stroke. Repeat until you feel your finger touch the LEGO^{®} plate or you visually see the first hole appearing in the eraser. Record the number of strokes.

**Table 11 -Back of Tile Test**

| Test Product | Water (g) | # Strokes To First Hole | Dry Eraser Weight (g) |
|---|---|---|---|
| MCME Original | 37.1 | 2 | 2.0 |
| MCME Original | 35.3 | 1 | 2.0 |
| MCME Original | 36.7 | 1 | 2.0 |
| MCME Original | 34.9 | 2 | 2.0 |
| MCME Original | 36.4 | 2 | 2.0 |
| MCME Original | 33.4 | 1 | 2.0 |
| MCME Original | 34.6 | 3 | 2.0 |
| MCME Original | 35.7 | 1 | 2.0 |
| MCME Original | 37.4 | 1 | 2.0 |
| MCME Original | 34.1 | 1 | 2.0 |
| MCME Extra Durable | 35.0 | 4 | 2.5 |
| MCME Extra Durable | 34.9 | 3 | 2.5 |
| MCME Extra Durable | 32.1 | 1 | 2.5 |
| MCME Extra Durable | 33.9 | 3 | 2.5 |
| MCME Extra Durable | 37.3 | 3 | 2.5 |
| MCME Extra Durable | 32.4 | 1 | 2.5 |
| MCME Extra Durable | 33.4 | 3 | 2.5 |
| MCME Extra Durable | 37.5 | 5 | 2.5 |
| MCME Extra Durable | 32.4 | 3 | 2.5 |
| MCME Extra Durable | 32.5 | 1 | 2.5 |
| Example 27 | 49.8 | 23 | 5.3 |
| Example 27 | 46.6 | 27 | 5.3 |
| Example 27 | 43.9 | 30 | 5.3 |
| Example 27 | 60.2 | 33 | 5.3 |
| Example 27 | 43.2 | 43 | 5.3 |
| Example 27 | 44.0 | 33 | 5.3 |
| Example 27 | 50.1 | 31 | 5.3 |
| Example 27 | 52.9 | 33 | 5.3 |
| Example 27 | 48.6 | 36 | 5.3 |
| Example 27 | 48.4 | 39 | 5.3 |
| UP&UP (Target) Original | 35.6 | 2 | 1.9 |
| UP&UP (Target) Original | 37.5 | 1 | 1.9 |
| UP&UP (Target) Original | 37.7 | 1 | 1.9 |
| UP&UP (Target) Original | 35.4 | 2 | 1.9 |
| UP&UP (Target) Original | 37.4 | 1 | 1.9 |
| UP&UP (Target) Extra Strength | 38.1 | 2 | 2.2 |
| UP&UP (Target) Extra Strength | 32.3 | 1 | 2.2 |
| UP&UP (Target) Extra Strength | 38.1 | 2 | 2.2 |
| UP&UP (Target) Extra Strength | 38.0 | 2 | 2.2 |
| UP&UP (Target) Extra Strength | 35.3 | 1 | 2.2 |
| Kroger Cleaning Solutions | 39.0 | 2 | 2.0 |
| Kroger Cleaning Solutions | 37.3 | 2 | 2.0 |
| Kroger Cleaning Solutions | 37.2 | 2 | 2.0 |
| Kroger Cleaning Solutions | 34.7 | 1 | 2.0 |
| Kroger Cleaning Solutions | 38.1 | 2 | 2.0 |
| Walmart Original | 36.1 | 1 | 1.7 |
| Walmart Original | 35.1 | 1 | 1.7 |
| Walmart Original | 35.0 | 1 | 1.7 |
| Walmart Original | 35.3 | 1 | 1.7 |
| Walmart Original | 36.4 | 1 | 1.7 |
| Walmart Extra Strength | 37.7 | 1 | 1.7 |
| Walmart Extra Strength | 38.7 | 1 | 1.7 |
| Walmart Extra Strength | 36.7 | 1 | 1.7 |
| Walmart Extra Strength | 35.4 | 2 | 1.7 |
| Walmart Extra Strength | 34.7 | 2 | 1.7 |

**Table 12 -LEGO^{®} Tile Test**

| Cleaning implement | Water (g) | # Strokes to First Hole | Dry Eraser Weight (g) |
|---|---|---|---|
| UP&UP (Target) Original | 34.0 | 4 | 1.9 |
| UP&UP (Target) Original | 37.4 | 4 | 1.9 |
| UP&UP (Target) Original | 33.6 | 5 | 1.9 |
| UP&UP (Target) Original | 33.9 | 4 | 1.9 |
| UP&UP (Target) Original | 37.2 | 6 | 1.9 |
| UP&UP (Target) Extra Strength | 38.5 | 2 | 2.2 |
| UP&UP (Target) Extra Strength | 37.8 | 4 | 2.2 |
| UP&UP (Target) Extra Strength | 31.5 | 2 | 2.2 |
| UP&UP (Target) Extra Strength | 37.0 | 4 | 2.2 |
| UP&UP (Target) Extra Strength | 36.0 | 3 | 2.2 |
| Kroger Cleaning Solutions Reusable Eraser Sponge (Original) | 36.5 | 7 | 2.0 |
| Kroger Cleaning Solutions Reusable Eraser | 34.7 | 6 | 2.0 |
| Sponge (Original) | | | |
| Kroger Cleaning Solutions Reusable Eraser Sponge (Original) | 34.0 | 6 | 2.0 |
| Kroger Cleaning Solutions Reusable Eraser Sponge (Original) | 38.1 | 6 | 2.0 |
| Kroger Cleaning Solutions Reusable Eraser Sponge (Original) | 35.0 | 5 | 2.0 |
| Walmart Original | 35.3 | 3 | 1.7 |
| Walmart Original | 34.9 | 3 | 1.7 |
| Walmart Original | 36.4 | 2 | 1.7 |
| Walmart Original | 36.8 | 3 | 1.7 |
| Walmart Original | 36.2 | 3 | 1.7 |
| Walmart Extra Strength | 32.4 | 3 | 1.7 |
| Walmart Extra Strength | 33.9 | 5 | 1.7 |
| Walmart Extra Strength | 36.6 | 2 | 1.7 |
| Walmart Extra Strength | 37.0 | 2 | 1.7 |
| Walmart Extra Strength | 36.3 | 2 | 1.7 |
| MCME Original | 39.5 | 5 | 2.0 |
| MCME Original | 38.1 | 1 | 2.0 |
| MCME Original | 37.0 | 3 | 2.0 |
| MCME Original | 39.4 | 3 | 2.0 |
| MCME Original | 34.3 | 3 | 2.0 |
| MCME Extra Durable | 32.0 | 5 | 2.5 |
| MCME Extra Durable | 32.4 | 3 | 2.5 |
| MCME Extra Durable | 32.8 | 6 | 2.5 |
| MCME Extra Durable | 36.2 | 5 | 2.5 |
| MCME Extra Durable | 35.3 | 8 | 2.5 |
| Example 27 | 58.1 | 67 | 5.3 |
| Example 27 | 65.1 | 88 | 5.3 |
| Example 27 | 61.9 | 72 | 5.3 |
| Example 27 | 67.6 | 61 | 5.3 |
| Example 27 | 62.3 | 85 | 5.3 |

**Table 13 - Averages for Back of Tile and LEGO^{®} Tests**

| Cleaning implement | Average # Strokes Back of Tile | Average # Strokes LEGO^{®} |
|---|---|---|
| MCME Original | 1.50 | 3.00 |
| MCME Extra Durable | 2.70 | 5.40 |
| Example 27 | 32.80 | 74.60 |
| UP&UP (Target) Original | 1.40 | 4.60 |
| UP&UP (Target) Extra Strength | 1.60 | 3.00 |
| Kroger Cleaning Solutions Reusable Eraser Sponge (Original) | 1.80 | 6.00 |
| Walmart Original | 1.00 | 2.80 |
| Walmart Extra Strength | 1.40 | 2.80 |

As is shown in Table 13, Example 27 has an increased average number of strokes until first hole for both back of tile and LEGO^{®} as compared to all of the comparative products. It is contemplated that the greater density and greater thickness provide an improved wet tensile strength as compared to the comparative products, leading to an increased number of strokes until first hole.

### Example 6: Pressure Distribution

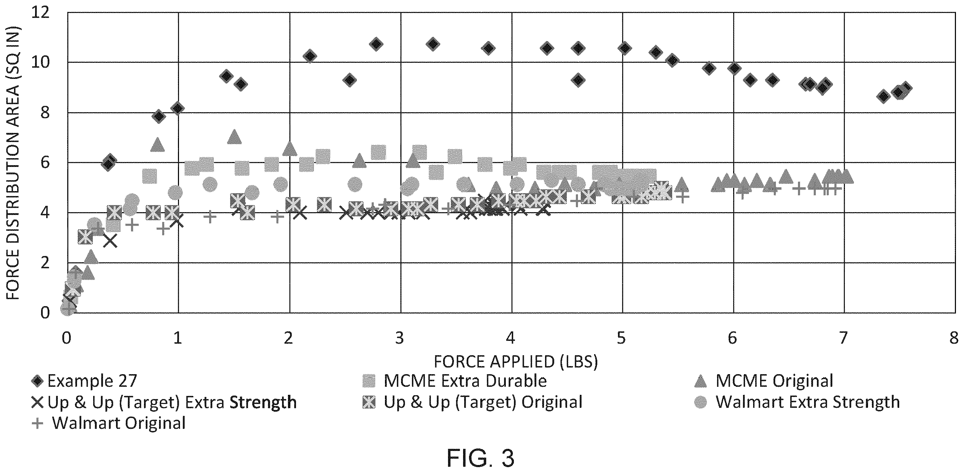

A Tekscan pressure mat model number 5315 was connected to a Tekscan sensor model number EH-2 sensor and a computer. The Tekscan Pressure Measurement System software I-Scan version 6.101 was opened. Then the equipment was calibrated for force and area. The cleaning implement sample was placed on the center of the pressure mat and slowly pressure was slowly applied with one finger in the center of the cleaning implement until reaching over 5 pounds (lbs) total force and then released. A chart, as shown in FIG. 3, was then produced showing the force distribution area (in square inches) over the total force applied (in pounds).

FIG. 3 shows that Example 27 exhibited an improved force distribution area per total force applied as compared to the comparative examples (MCME Original, MCME Extra Durable, UP&UP (Target) Original, UP&UP (Target) Extra Strength, Walmart Original, and Walmart Extra Strength) at least after about 0.5, 0.75, or 1 pound(s) of force applied. In particular, Example 27 cleaning implement exhibited a force distribution area of greater than or equal to about 6 square inches (in²) at a total force applied of greater than or equal to about 0.5 pounds and consistently performed with a greater force distribution area as compared to the comparative examples when total force applied was greater than or equal to about 0.5 pounds. Additionally, Example 27 cleaning implement exhibited a force distribution area of greater than or equal to about 7.5 square inches (in²) at a total force applied of greater than or equal to about 1 pound and consistently performed with a greater force distribution area as compared to the comparative examples when total force applied was greater than or equal to about 1 pound.

The MCME Extra Durable tapered out at a maximum force distribution area of about 6 in² after about 1 or 1.5 pounds. Additionally, MCME Original reached a maximum force distribution area of about 7 in² at about 1.5 pounds of force applied, and then MCME Original consistently decreased in force distribution area as more force was applied. In contrast, Example 27 had a maximum force distribution area of from about 9 to 11 in² from about 1.5 pounds to 6.5 pounds. Without intending to be bound by theory, it is contemplated that the cleaning implement according to the present disclosure exhibited improved force distribution area over total force applied when compared with the comparative examples due to the increased thickness of Example 27.

Every document cited herein, including any cross referenced or related patent or application, is hereby incorporated herein by reference in its entirety unless expressly excluded or otherwise limited. The citation of any document is not an admission that it is prior art with respect to any embodiment disclosed or claimed herein or that it alone, or in any combination with any other reference or references, teaches, suggests or discloses any such embodiment. Further, to the extent that any meaning or definition of a term in this document conflicts with any meaning or definition of the same term in a document incorporated by reference, the meaning or definition assigned to that term in this document shall govern.

For the purposes of defining the present technology, the transitional phrase "consisting of" may be introduced in the claims as a closed preamble term limiting the scope of the claims to the recited components or steps and any naturally occurring impurities. For the purposes of defining the present technology, the transitional phrase "consisting essentially of' may be introduced in the claims to limit the scope of one or more claims to the recited elements, components, materials, or method steps as well as any non-recited elements, components, materials, or method steps that do not materially affect the novel characteristics of the claimed subject matter. The transitional phrases "consisting of" and "consisting essentially of" may be interpreted to be subsets of the open-ended transitional phrases, such as "comprising" and "including," such that any use of an open ended phrase to introduce a recitation of a series of elements, components, materials, or steps should be interpreted to also disclose recitation of the series of elements, components, materials, or steps using the closed terms "consisting of" and "consisting essentially of." For example, the recitation of a composition "comprising" components A, B, and C should be interpreted as also disclosing a composition "consisting of" components A, B, and C as well as a composition "consisting essentially of" components A, B, and C. Any quantitative value expressed in the present application may be considered to include open-ended embodiments consistent with the transitional phrases "comprising" or "including" as well as closed or partially closed embodiments consistent with the transitional phrases "consisting of' and "consisting essentially of."

As used in the Specification and appended Claims, the singular forms "a", "an", and "the" include plural references unless the context clearly indicates otherwise. The verb "comprises" and its conjugated forms should be interpreted as referring to elements, components or steps in a non-exclusive manner. The referenced elements, components or steps may be present, utilized or combined with other elements, components or steps not expressly referenced.

It should be understood that any two quantitative values assigned to a property may constitute a range of that property, and all combinations of ranges formed from all stated quantitative values of a given property are contemplated in this disclosure. The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm".

The subject matter of the present disclosure has been described in detail and by reference to specific embodiments. It should be understood that any detailed description of a component or feature of an embodiment does not necessarily imply that the component or feature is essential to the particular embodiment or to any other embodiment.

It should be apparent to those skilled in the art that various modifications and variations may be made to the embodiments described within without departing from the spirit and scope of the claimed subject matter. Thus, it is intended that the specification cover the modifications and variations of the various embodiments described within provided such modifications and variations come within the scope of the appended claims and their equivalents. Unless otherwise stated within the application, all tests, properties, and experiments are conducted at room temperature and atmospheric pressure.

Having described the subject matter of the present disclosure in detail and by reference to specific embodiments thereof, it is noted that the various details disclosed within should not be taken to imply that these details relate to elements that are essential components of the various embodiments described within, even in cases where a particular element is illustrated in each of the drawings that accompany the present description. Further, it should be apparent that modifications and variations are possible without departing from the scope of the present disclosure, including, but not limited to, embodiments defined in the appended claims. More specifically, although some aspects of the present disclosure are identified as particularly advantageous, it is contemplated that the present disclosure is not necessarily limited to these aspects.

## Claims

1. A consumer product comprising:
a first fragrance material that is a *meta-(*C1-C4 alkoxy)salicylaldehyde, wherein the first fragrance material is present in an amount of at least 0.0005%, by weight of the consumer product composition, and
a treatment adjunct.

2. A consumer product comprising:
a first fragrance material that is a *meta-(*C1-C4 alkoxy)salicylaldehyde,
an optional second fragrance material selected from the group consisting of vanillin, ethylvanillin, or a combination thereof,
wherein the weight ratio of the first fragrance material to the second fragrance material, if present, is from 100:0 to 1:99; and
a treatment adjunct.

3. The consumer product according to any of claims 1 or 2, wherein the first fragrance material is a *meta-(*C1-C2 alkoxy)salicylaldehyde.

4. The consumer product according to any preceding claim, wherein the first fragrance material is a *meta-(*C2 alkoxy)salicylaldehyde.

5. The consumer product according to any preceding claim, wherein the alkoxy group in the first fragrance material is a linear alkoxy group.

6. The consumer product according to any preceding claim, wherein the first fragrance material is present in the consumer product in an amount of at least 0.001%, preferably at least 0.01%, more preferably at least 0.1%, more preferably at least 0.5%, more preferably at least 1%, by weight of the consumer product.

7. The consumer product according to any preceding claim, wherein the first fragrance material is synthetically prepared.

8. The consumer product according to any preceding claim, wherein the first fragrance material is part of a perfume delivery system, where the perfume delivery system is a delivery particle, a pro-fragrance material, or a combination thereof.

9. The consumer product according to any of claims 2-8, wherein the weight ratio of the first fragrance material to the second fragrance material, if present, is from 100:0 to 25:75, preferably from 100:0 to 50:50, more preferably from 100:0 to 51:49, more preferably from 100:0 to 75:25, more preferably from 100:0 to 90:10, more preferably from 100:0 to 95:5, more preferably from 100:0 to 99:1.

10. The consumer product according to any preceding claim, wherein the consumer product is substantially free of a second fragrance material selected from the group of vanillin, ethylvanillin, and a combination thereof.

11. The consumer product according to any preceding claim, wherein the treatment adjunct is selected from surfactants, conditioning actives, deposition aids, rheology modifiers or structurants, bleach systems, stabilizers, builders, chelating agents, dye transfer inhibiting agents, dispersants, enzymes, enzyme stabilizers, catalytic metal complexes, polymeric dispersing agents, clay and soil removal/anti-redeposition agents, brighteners, suds suppressors, silicones, hueing agents, aesthetic dyes, neat perfume, additional perfume delivery systems, structure elasticizing agents, carriers, hydrotropes, processing aids, anti-agglomeration agents, coatings, formaldehyde scavengers, pigments, or mixtures thereof.

12. The consumer product according to any preceding claim, wherein the treatment adjunct comprises a carrier,
preferably a carrier comprising isopropyl myristate (IPM), dipropylene glycol, propylene glycol monomethyl ether (DPM), tripropyleneglycol monomethyl ester (TPM), triethyl citrate (TEC), or a mixture thereof,
more preferably a carrier comprising isopropyl myristate.

13. The consumer product according to any preceding claim, wherein the consumer product is a baby care product, a beauty care product, a fabric care product, a home care product, a family care product, a feminine care product, a health care product, a food product, a beverage product, a fine fragrance product, or a combination thereof.

14. The consumer product according to any preceding claim, wherein the consumer product is a fabric care product and/or a home care product,
wherein the home care product is selected from the group consisting of a hard surface cleaner, a dish care product, an air care product, or a combination thereof.

15. The consumer product according to any preceding claim, wherein the consumer product comprises a form selected from a liquid composition, a granular composition, a hydrocolloid, a single-compartment pouch, a multi-compartment pouch, a dissolvable sheet, a pastille or bead, a fibrous article, a tablet, a stick, a bar, a flake, a spray, a foam/mousse, a non-woven sheet, a cream, an absorbent article, a paste, an adhesive, an adhesive patch, or a mixture thereof.
